# EUROPÄISCHE PATENTSCHRIFT

(11) **EP 1 951 343 B1**
(45) Veröffentlichungstag und Bekanntmachung des Hinweises auf die Patenterteilung: **15.09.2010**
(21) Anmeldenummer: 06828997.4
(22) Anmeldetag: 10.11.2006
(51) Int. Cl.: A61M 5/32, A61M 5/34

(54) **AUFSATZ FÜR EINE SPRITZE ODER KARPULE**
ATTACHMENT FOR A SYRINGE OR CARTRIDGE
CHAPEAU POUR UNE SERINGUE OU POUR UNE CARPULE

(30) Priorität: 12.11.2005 DE 102005054075
(43) Veröffentlichungstag der Anmeldung: 06.08.2008
(73) Patentinhaber: Arzneimittel GmbH Apotheker Vetter & Co. Ravensburg, 88212 Ravensburg (DE)
(72) Erfinder: HUND, Petra, 88276 Berg (DE); GLOCKER, Joachim, 88250 Weingarten (DE); SCHWARZ, Walter, 88339 Bad Waldsee (DE)
(74) Vertreter: Gleiss, Alf-Olav
(86) Internationale Anmeldenummer: PCT/EP2006/010791
(87) Internationale Veröffentlichungsnummer: WO 2007/054333

(56) Entgegenhaltungen:
- EP-A- 1 502 616
- EP-A2- 0 873 758
- WO-A-2006/090118
- DE-A1- 2 254 153
- DE-A1- 19 954 373
- DE-C1- 4 140 101
- FR-A1- 2 522 971
- GB-A- 868 134
- GB-A- 933 587
- GB-A- 1 540 881
- US-A- 2 731 012
- US-A- 2 799 272

## Beschreibung

Die Erfindung betrifft einen Aufsatz für eine Spritze oder Karpule gemäß Oberbegriff des Anspruchs 1 sowie einen Aufsatz für eine Spritze oder Karpule gemäß Oberbegriff des Anspruchs 2, außerdem ein Verfahren zur Herstellung eines Aufsatzes für eine Spritze oder Karpule gemäß Anspruch 15, ein Verfahren zur Herstellung eines Aufsatzes für eine Spritze oder Karpule gemäß Oberbegriff des Anspruchs 16, ein Verfahren zur Herstellung eines Aufsatzes für eine Spritze oder Karpule gemäß Oberbegriff des Anspruchs 17, außerdem eine Spritze oder Karpule gemäß Anspruch 20 und schließlich eine Spritze oder Karpule gemäß Anspruch 21.

Aufsätze und deren Herstellung sowie Spritzen oder Karpulen mit Aufsätzen sind bekannt (GB 1 540 881 A, EP 0 873 758 A2). Die Aufsätze weisen ein Dichtelement auf, das dazu dient, die Spritze oder Karpule im Bereich einer Kanüle so dicht abzuschließen, dass deren Inhalt über längere Zeit gelagert werden kann, ohne dass es zu Kontaminationen, insbesondere durch Bakterien, Viren oder dergleichen kommt. Es hat sich herausgestellt, dass in vielen Fällen sich das Material des Dichtelements im Auflagebereich auf der Spritze oder Karpule so verändert, dass eine mikrobiologische Dichtigkeit nicht gewährleistet werden kann.

Aufgabe der Erfindung ist es daher, einen Aufsatz zu schaffen, der diese Nachteile nicht aufweist.

Zur Lösung dieser Aufgabe wird ein Aufsatz geschaffen, der die in Anspruch 1 genannten Merkmale aufweist. Er umfasst ein Dichtelement, das die Kanüle abschließt, wobei das Dichtelement auf die Kanüle aufgespritzt ist. Der Aufsatz zeichnet sich dadurch aus, dass eine Kappe vorgesehen ist, die auf das Dichtelement aufspritzbar ist. Durch das Aufspritzen des Dichtelements ist ausgeschlossen, dass die Kanüle beschädigt oder verbogen wird. Es ist damit gewährleistet, dass sie immer optimal von dem Dichtelement umfasst wird. Außerdem wird durch das Aufspritzen des Dichtelements eine Verbindung mit der Kanüle geschaffen. Die Kappe, welche auf das Dichtelement aufspritzbar ist, dient als Schutzkappe, so dass eine mechanische Beschädigung der Kanüle während der Lagerzeit sicher vermieden wird. Das Aufspritzen der Kappe auf das Dichtelement gewährleistet eine besonders einfache Verbindung zwischen diesen Elementen, sowie eine einfache Herstellung. Insgesamt ist es damit möglich, eine besonders gute Abdichtung des Auslasses der Kanüle zu gewährleisten, so dass auch bei einer Langzeitlagerung der Spritze oder Kanüle eine mikrobiologische Verunreinigung von deren Inhalt verhindert wird.

Zur Lösung dieser Aufgabe wird auch ein Aufsatz vorgeschlagen, der die Merkmale des Anspruchs 2 aufweist und für eine Spritze oder Karpule mit einem Grundkörper und mit einem Ansatz zur Befestigung des Aufsatzes vorgesehen ist, und der außerdem eine Kappe sowie ein Dichtelement aufweist. Das Dichtelement und die Kappe sind durch ein Spritzgussverfahren miteinander verbunden. Der Aufsatz zeichnet sich dadurch aus, dass ein Kanülen-Hub vorgesehen ist, in den eine Kanüle eingesetzt ist, und der Gummi oder TPE umfasst oder aus Gummi oder TPE besteht. Diese Materialien gewähren eine optimale Abdichtung der Spritze oder Karpule im Bereich der Kanüle auch über eine lange Lagerzeit. Weitere Ausgestaltungen ergeben sich aus den Unteransprüchen.

Zur Lösung dieser Aufgabe wird auch ein Verfahren zur Herstellung eines Aufsatzes für eine Spritze oder eine Karpule mit den Merkmalen des Anspruchs 15 vorgeschlagen, das die folgenden Schritte aufweist: eine Kanüle wird an ihrem Ende erfasst, das nicht zur Injektion dient. Auf das dann freie Ende, das also zur Injektion dient, wird ein Dichtelement aufgespritzt. Das Verfahren zeichnet sich dadurch aus, dass das Dichtelement mit einer Kappe umspritzt wird. Durch das Umspritzen des zur Injektion dienenden Endes der Kanüle wird gewährleistet, dass dieses besonders dicht verschlossen ist, so dass eine Kontamination auch bei Langzeitlagerung ausgeschlossen ist. Weiterhin ist auch eine Deformation oder Beschädigung der Kanüle beim Umspritzen mit dem Dichtelement ausgeschlossen. Das Umspritzen des Dichtelements mit einer Schutzkappe gewährleistet den Schutz desselben vor mechanischer Beschädigung auch über eine lange Zeit. Außerdem ist das Verfahren aufgrund der zweimaligen Anwendung eines Spritzgussverfahrens besonders einfach und kostengünstig realisierbar. Zur Lösung dieser Aufgabe wird auch ein Verfahren mit den Merkmalen des Anspruchs 16 zur Herstellung eines Aufsatzes für eine Spritze oder eine Karpule, der nach Anspruch 1 ausgebildet ist, vorgeschlagen. Das Verfahren zeichnet sich dadurch aus, dass das Dichtelement und die Kappe durch ein Spritzgussverfahren miteinander verbunden sind. Hierbei kann eine sichere Verbindung zwischen Kappe und Dichtelement gewährleistet werden, so dass ein Langzeitschutz gegen mechanische Beschädigung des Dichtelements gegeben ist. Außerdem können Dichtelement und Kappe beziehungsweise insbesondere deren Verbindung sehr einfach und kostengünstig realisiert werden.

Zur Lösung dieser Aufgabe wird auch ein Verfahren mit den Merkmalen des Anspruchs 17 zur Herstellung eines Aufsatzes für eine Spritze oder eine Karpule, der gemäß Anspruch 2 ausgebildet ist, vorgeschlagen. Das Dichtelement und die Kappe werden mittels eines Spritzgussverfahrens miteinander verbunden, also besonders einfach und kostengünstig. Gleichzeitig wird gewährleistet, dass das Dichtelement die Spritze oder Karpule besonders gut abschließt und vor Verunreinigungen schützt. Das Verfahren zeichnet sich dadurch aus, dass bei dem Aufsatz ein Kanülen-Hub vorgesehen ist, in den eine Kanüle eingesetzt ist, und der Gummi oder TPE umfasst, oder aus Gummi oder TPE besteht. Diese Materialien gewährleisten eine besonders gute Abdichtung im Bereich der Kanüle, so dass eine Kontamination auch während einer Langzeitlagerung sicher ausgeschlossen werden kann. Weitere Ausführungsformen des Verfahrens ergeben sich aus den Unteransprüchen.

Zur Lösung dieser Aufgabe wird außerdem eine Spritze oder Karpule gemäß Anspruch 20 vorgeschlagen, die einen Aufsatz der oben genannten Art umfasst. Die Spritze oder Karpule zeichnet sich dadurch aus, dass deren Inhalt besonders gut gegen Verunreinigungen geschützt ist.

Schließlich wird zur Lösung dieser Aufgabe eine Spritze oder Karpule gemäß Anspruch 21 vorgeschlagen, die einen Aufsatz aufweist, der nach einem der oben genannten Verfahren hergestellt ist.

Die Erfindung wird im Folgenden anhand der Zeichnung näher erläutert. Es zeigen:
- Figur 1: eine perspektivische Ansicht eines ersten Ausfüh- rungsbeispiels eines Aufsatzes;
- Figur 2: einen Längsschnitt durch den in Figur 1 dargestellten Aufsatz;
- Figur 3: einen Längsschnitt durch ein zweites Ausführungsbei- spiel eines Aufsatzes;
- Figur 4: ein drittes Ausführungsbeispiel eines Aufsatzes in drei Montagepositionen;
- Figur 5: eine Seitenansicht eines abgewandelten Ausführungs- beispiels eines Aufsatzes;
- Figur 6: einen Längsschnitt durch den Aufsatz gemäß Figur 5;
- Figur 7: eine perspektivische Ansicht eines nicht zur Erfindung gehörenden Aufsatzes;
- Figur 8: einen Längsschnitt durch den Aufsatz gemäß Figur 7;
- Figur 9: eine perspektivische Ansicht eines weiteren nicht zur Erfindung gehörenden Aufsatzes und
- Figur 10: einen Längsschnitt durch den Aufsatz gemäß Figur 9.

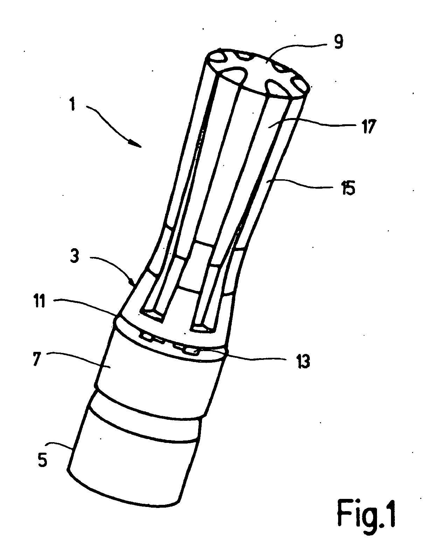

Das in Figur 1 in perspektivischer Ansicht dargestellte Ausführungsbeispiel eines auch als Kanülenaufsatz bezeichneten Aufsatzes 1 umfasst eine als Schutzkappe dienende Kappe 3, die auf eine Spritze 5 aus Kunststoff oder vorzugsweise Glas aufgesetzt ist. Die Kappe 3 ist zweiteilig ausgebildet und umfasst einen unmittelbar an der Spritze 5 befestigten Haltebereich 7 sowie ein Kappenelement 9. Die beiden Teile sind über eine Sollbruchlinie 11 miteinander verbunden, die grundsätzlich bekannt ist und dazu dient, dass das Kappelelement 9 auf einfache Weise vom Haltebereich 7 und damit von der Spritze 5 getrennt werden kann. Die Sollbruchlinie wird von Haltestegen 13 überspannt, die das Kappenelement 9 und den Haltebereich 7 miteinander verbinden, aber so schwach ausgebildet sind, dass das Kappenelement 9 durch eine Kipp-, Zieh- oder Drehbewegung vom Haltebereich 7 getrennt werden kann. Dadurch wird eine unter der Kappe 3 liegende Kanüle freigelegt. Bei dem ersten Öffnen wird die Sollbruchlinie 11 aufgesprengt, sodass Manipulationen ohne weiteres feststellbar sind. Die Klappe 3 dient somit als Originalitätssicherung.

Das Kappenelement 9 weist mehrere in dessen Längsrichtung verlaufende Stege 15 auf, die in einem Abstand zueinander angeordnet sind und die ein auch als Schutzkappe bezeichnetes erstes Dichtelement 17 halten. Dieses besteht vorzugsweise aus Gummi oder aus TPE, also einem thermoplastischen Elastomer. Es ist besonders griffig und schließt das freie Ende der hier nicht sichtbaren Kanüle ab. Das erste Dichtelement 17 wird an die Kanüle angeformt, vorzugsweise angespritzt. Die Kappe 3 kann dann auf das erste Dichtelement 17 aufgesetzt werden. Es wird bevorzugt, die Kappe 3 auf das Dichtelement 17 aufzuspritzen, also in einem Spritzgussverfahren herzustellen, um eine Verbindung zwischen Kappe 3 und Dichtelement 17 zu schaffen. Die Kappe 3 selbst kann grundsätzlich auch Gummi oder TPE umfassen oder aus einem oder beiden der genannten Materialien hergestellt werden. In der Regel weist sie aber Hartkunststoff auf oder besteht vorzugsweise aus diesem Material. Vorzugsweise werden die Kappe 3 und das erste Dichtelement 17 in einem Zwei-Komponenten-Spritzgussverfahren hergestellt.

Besonders bevorzugt wird ein Ausführungsbeispiel des Aufsatzes 1, bei dem das Dichtelement 17 im Berührungsbereich mit der Spritze oder Karpule TPE oder Gummi aufweist beziehungsweise aus TPE oder Gummi besteht, um eine optimale Abdichtung der Spritze beziehungsweise Karpule zu gewährleisten. Diese Materialien haben die Eigenschaft, dass sie auch bei einer längeren Lagerung der Spritze oder Karpule nicht fließen und damit eine mikrobiologisch einwandfreie Abdichtung der Spritze beziehungsweise Karpule sicherstellen. Die Kappe 3 besteht vorzugsweise aus Hartkunststoff und schützt damit das Dichtelement 17 bei Lagerung und Transport der Spritze beziehungsweise Karpule. Besonders einfach ist das Entfernen der Kappe 3, wenn diese eine Sollbruchlinie 11 aufweist. Letztlich kann aber auf diese auch verzichtet und der Aufsatz 1 als Ganzes von einer Spritze oder Karpule entfernt werden.

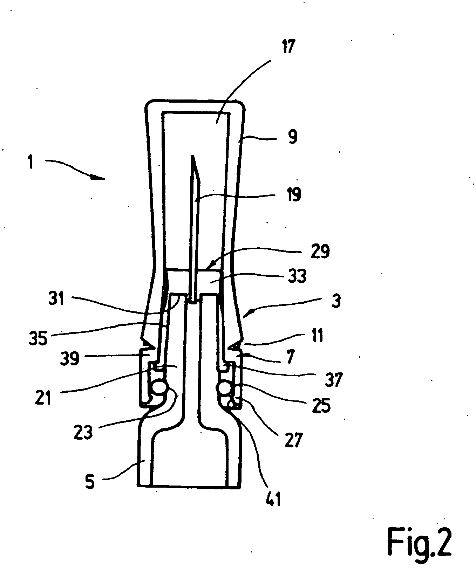

Das erste Ausführungsbeispiel des Aufsatzes 1 ist in Figur 2 im Längsschnitt dargestellt. Gleiche Teile sind mit gleichen Bezugsziffern versehen, sodass insofern auf die vorangegangene Figur verwiesen wird.

Deutlich erkennbar ist hier die Kappe 3 mit dem Kappenelement 9 und dem Haltebereich 7, der über die Sollbruchlinie 11 mit dem Kappenelement 9 verbunden ist.

Deutlich wird hier, dass die Kanüle 19 im ersten Dichtelement 17 angeordnet ist, das als Nadelschutz wirkt und dient.

Die Schnittdarstellung gemäß Figur 2 zeigt, dass die Spritze 5 einen hier als Luer-Ansatz ausgebildeten Ansatz 21 aufweist, auf den der Aufsatz 1 aufgesetzt wird.

Der Ansatz 21 kann eine Ringnut 23 aufweisen, in die ein Haltering 25 eingreift, der damit sicher -in axialer Richtung des Ansatzes 21 gesehen- gehalten wird. Der Haltebereich 7 übergreift den Haltering 25 und wird an diesem mittels mindestens einer Rastnase 27 sicher gehalten. Anstelle der mindestens einen Rastnase 27 kann auch ein geschlossener nach innen vorspringender Rastring vorgesehen werden.

Die Kanüle 19 ist in einen auch als Kanülenhalter bezeichneten Kanülen-Hub 29 eingeformt, der eine zwischen dem ersten Dichtelement 17 und der Stirnseite 31 des Ansatzes 21 liegende Grundplatte 33 aufweist, die den Boden des im Wesentlichen napfförmig ausgebildeten Kanülen-Hubs bildet und von der eine zylindrische oder leicht konische Wand 35 ausgeht. An dem der Grundplatte 33 abgewandten Ende der Wand 35 ist ein umlaufender, nach außen stehender Rand 37 vorgesehen, der unter einer umlaufenden Schulter 39 zu liegen kommt, die vom Haltebereich 7 radial nach innen vorspringt und damit den Rand 37, also den Kanülen-Hub 29 sicher hält.

Von der Spritze 5 aus gesehen ist die Sollbruchlinie 11 oberhalb der Schulter 39 angeordnet, sodass beim Abbrechen, -drehen oder -ziehen des Kappenelements 9 vom Haltebereich 7 der Kanülen-Hub 29 immer noch sicher an der Spritze 5 gehalten wird, weil die Rastnase 27 auch dann noch fest unterhalb des Halterings 25 verankert ist

Die Darstellung zeigt, dass die Rastnase 27 -oder eben der Rastringeine nach innen weisende Anlaufschräge 41 aufweist, sodass der Aufsatz 1 leicht auf die Spritze 5 aufgesetzt werden kann, wobei dann die Rastnase 27 unterhalb des Halterings 25 einschnappt.

Der Kanülen-Hub 29 weist vorzugsweise Gummi oder TPE auf, insbesondere ist er aus Gummi oder TPE hergestellt, weil auf diese Weise eine optimale Abdichtung der Spritze 5 gewährleistet ist.

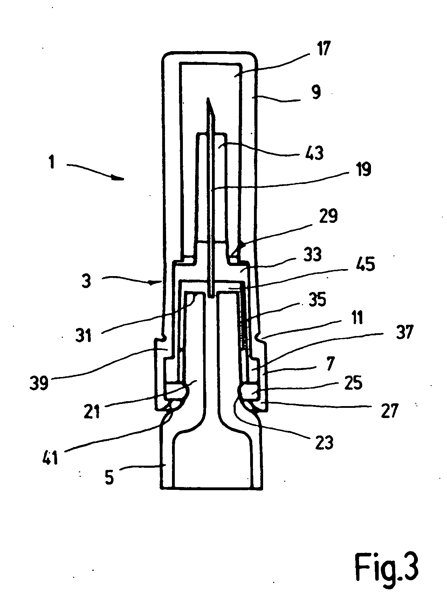

Figur 3 zeigt einen Längsschnitt durch ein abgewandeltes Ausführungsbeispiel eines Aufsatzes 1. Gleiche Teile sind mit gleichen Bezugsziffern versehen, sodass insofern auf die Beschreibung zu den vorangegangenen Figuren verwiesen wird.

Der Längsschnitt zeigt, dass auf die Spritze 5 wiederum eine Kappe 3 aufgesetzt ist, die einen der Spritze 5 zugewandten Haltebereich 7 und ein über eine Sollbruchlinie 11 mit diesem verbundenes Kappenelement 9 aufweist, in dem ein erstes Dichtelement 17 vorgesehen ist. Dieses umschließt hier einen Hohlraum 43, der sich nicht über die gesamte Länge der aus dem Kanülen-Hub 29 vorstehenden Kanüle 19 erstreckt. Deren dem Kanülen-Hub 29 abgewandtes Ende liegt aber innerhalb des ersten Dichtungselements 17.

Aus der Darstellung ist auch ersichtlich, dass der Kanülen-Hub nicht unmittelbar auf der Stirnseite 31 des Ansatzes 21 anliegt. Vielmehr ist hier ein zweites Dichtelement 45 vorgesehen, das sich wie eine Kappe über den an die Stirnseite 31 angrenzenden Bereich des Ansatzes 21 erstreckt.

Vorzugsweise umfasst das zweite Dichtelement 45 Gummi oder TPE oder ist aus Gummi beziehungsweise TPE hergestellt. Durch die unmittelbare Anlage des elastischen Materials des zweiten Dichtelements 45 an der Spritze 5 ergibt sich eine besonders gute Abdichtung der Spritze 5. Grundsätzlich kann auch Hartkunststoff für das zweite Dichtelement 45 verwendet werden. Da dieses Material sich jedoch verändert, also fließt, sind die Dichtungseigenschaften nicht optimal. Deshalb wird hier ein Aufsatz 1 vorgeschlagen, bei dem ein Produkt in der Spritze 5 oder Karpule während der Lagerung nur mit Glas oder mit Stahl in Berührung kommt, oder aber mit dem zweiten Dichtelement 45 aus Gummi oder TPE.

Da hier das zweite Dichtelement 45 elastisch ist und eine optimale Dichtung der Spritze 5 gewährleistet, kann der Kanülen-Hub 29 ohne weiteres aus Hartkunststoff hergestellt werden.

Der Kanülen-Hub 29 weist hier eine Grundplatte 33 und eine davon ausgehende umlaufende Wand 35 auf, die an ihrem der Grundplatte 33 abgewandten Ende einen Rand 37 umfasst, der von einer umlaufenden Schulter 39 des Haltebereichs 7 gehalten wird. Anstelle der umlaufenden Schulter 39 kann hier, wie bei dem Ausführungsbeispiel nach Figur 2, vorgesehen werden, dass lediglich einzelne Vorsprünge zur Fixierung des Kanüfen-Hubs 29 vorgesehen werden.

Auch hier ist der Ansatz 21 mit einer umlaufenden Ringnut 23 versehen, in die ein Haltering 25 eingreift. Die Kappe 3 wird auch hier von mindestens einer Rastnase 27 oder von einem umlaufenden Rastring am Haltering 25 gehalten.

Auch hier ist vorgesehen, dass die Rastnase 27 eine Anlaufschräge 41 aufweist, sodass das Aufsetzen des Aufsatzes 1 erleichtert wird. Bei den hier gezeigten Ausführungsbeispielen wurde ein separater Haltering 25 beschrieben. Es ist sehr wohl möglich, diesen in den Kanülen-Hub 29 zu integrieren, also den Haltering 25 und den Kanülen-Hub 29 aus einem Stück herzustellen. Der untere, der Spritze 5 zugewandte Rand des Kanülen-Hubs 29 ist in diesem Fall mit einem nach innen vorspringenden, als Haltering 25 wirkenden Ringwulst versehen.

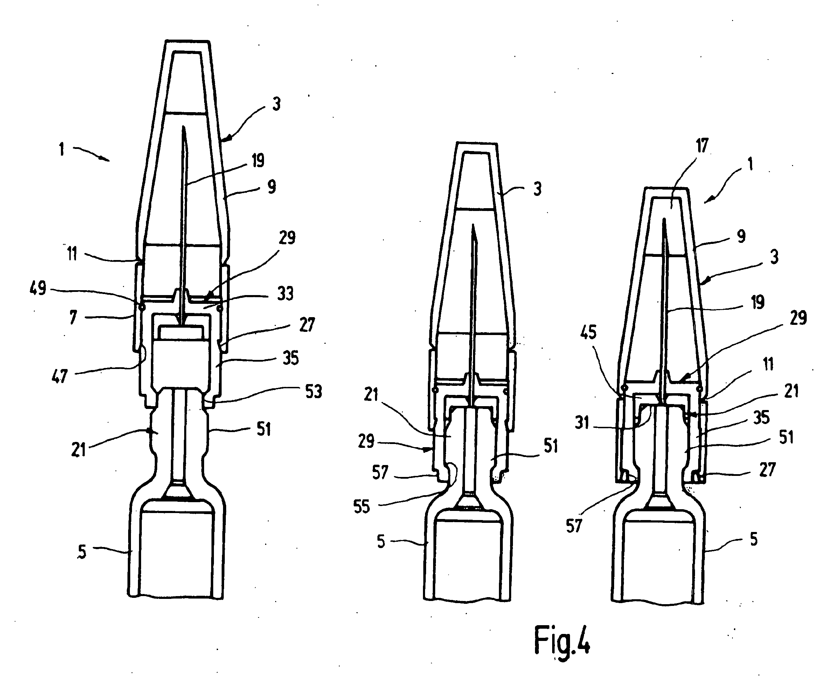

Die Figur 4 zeigt ein weiteres Ausführungsbeispiel eines Aufsatzes 1 in drei unterschiedlichen Montagepositionen. Gleiche Teile sind mit gleichen Bezugsziffern versehen, so dass insofern auf die vorangegangene Beschreibung verwiesen wird.

Der Aufsatz 1 weist eine Kappe 3 auf, die ein Kappenelement 9 und einen über eine Sollbruchlinie 11 damit verbundenen Haltebereich 7 umfasst. Eine Kanüle 19 ist in einen Kanülen-Hub 29 eingeformt, vorzugsweise eingespritzt, der auch hier eine Grundplatte 33 aufweist, von der eine vorzugsweise umlaufende Wand 35 ausgeht.

Der Haltebereich 7 weist an seinem unteren, der Spritze 5 zugewandeten Randbereich mindestes eine Rastnase 27, vorzugsweise einen umlaufenden radial nach innen entspringendem Rastring auf, der in eine auf der Außenfläche des Kanülen-Hubs 29 vorgesehenen Ringnut 47 eingreift. Zusätzlich kann zwischen der Innenseite des Haltebereichs 7 und der Außenfläche des Kanülenhubs 29 eine Dichtungseinrichtung vorgesehen werden, die hier als O-Ring 49 ausgebildet ist. Dieser schützt die Kanüle 19 während der Lagerung.

Der Aufsatz 1 ist bei der linken Darstellung in Figur 1 auf den Ansatz 21 der Spritze 5 aufgesetzt, der hier mindestens einen radial nach außen vorspringenden Vorsprung vorzugsweise einen Ringwulst 51 aufweist, dessen Außendurchmesser größer ist, als der Innendurchmesser der Wandung 35 an ihrem der Grundplatte 33 abgewandtem Ende, wo eine Haltevorrichtung für den Kanülen-Hub 29 ausgebildet ist, hier ein nach innen vorspringender, vorzugsweise ringförmiger Vorsprung 53.

In der zweiten, in Figur 4 mittleren Montagestellung ist der Konusaufsatz 1 nach unten in Richtung auf die Spritze 5 gedrückt worden, so dass der Kanülen-Hub 29 den Ringwulst 51 umgreift, wobei der Vorsprung 53 unterhalb des Ringwulstes 51 einrastet, also an dessen der Spritze 5 zugewandten Außenschulter 55. Der Vorsprung 53 wirkt damit wie ein Haltering, der in eine unterhalb des Ringwulstes 51 liegende Nut eingreift. Aus der Darstellung wird deutlich, dass in dieser zweiten Montageposition noch keine Relativbewegung zwischen der Kappe 3 und dem Kanülen-Hub erfolgt ist.

Die dritte Montageposition ist ganz rechts in Figur 4 dargestellt. Hier ist der Aufsatz 1 fertig auf der Spritze 5 montiert, indem die Kappe 3 ganz nach unten verlagert wurde, also in Richtung auf die Spritze 5, so dass mindestens die Rastnase 27 des Haltebereichs 7 der Kappe 3 unter dem Kanülen-Hub 29 einrastet. Hier ist auf der Außenseite des Haltebereich eine entsprechende Ringnut 57 vorgesehen, in die die Rastnase 27 eingreifen kann.

In dieser Montageposition umgreift der Haltebereich 7 die Wand 35 des Kanülen-Hubs 29 von außen und sichert damit den Kanülen-Hub 29 an der Außenschulter 55 des Ansatzes 21, so dass Manipulationen am Konusaufsatz 1 für einen Benutzer ohne weiteres sichtbar werden: Übt ein Benutzer auf das Kappenelement 9 eine seitliche Kraft aus, oder eine Zug- oder eine Drehkraft, so bricht die Sollbruchlinie 11, bzw. die dort vorgesehenen Haltestege 13, die hier nicht dargestellt sind. Damit löst sich das Kappenelement 9 vom Halteelement 7, was für einen Benutzer ohne weiteres erkennbar ist. Es wird hier also eine Originalitäts- oder Manipulationssicherung realisiert.

In dem Kappenelement 9 befindet sich in dessen Innenraum an dem dem Kanülen-Hub 29 abgewandten Ende ein erstes Dichtelement 17. Dieses erstreckt sich -in Längsrichtung des Kappenelements 9 gesehen- in einen Bereich, der so gewählt ist, dass in der dritten Montageposition die Kanüle 19 im ersten Dichtelement 17 eingebettet ist.

Das erste Dichtelement 17 umfasst Gummi oder TPE oder ist aus Gummi oder TPE hergestellt. Die Kappe 3 kann ebenfalls diese Materialien umfassen oder aus diesen hergestellt sein. Vorzugsweise ist sie jedoch aus Hartkunststoff hergestellt, um dem Aufsatz 1 mehr Stabilität zu verleihen und das Dichtelement zu schützen.

Der Kanülen-Hub 29 kann Gummi oder TPE umfassen, oder aber aus einem oder beiden dieser Materialien bestehen, um eine optimale Abdichtung gegenüber dem Ansatz 21 der Spritze 5 zu gewährleisten. Eine derartige Ausgestaltung wurde anhand von Figur 2 erläutert.

Wie anhand des Ausführungsbeispiels gemäß Figur 3 erläutert, kann zwischen dem Kanülen-Hub 29 und der Stirnseite 31 des Ansatzes 21 ein zweites Dichtelement 45 vorgesehen werden. Dieses ist in Figur 4 dargestellt. Vorzugsweise ist das zweite Dichtelement 45 optimal auf die Abdichtung zwischen Aufsatz 1 und Spitze 5 ausgelegt und weist daher Gummi oder TPE auf, oder ist aus Gummi oder TPE hergestellt.

Bei dem in Figur 4 dargestelltem Ausführungsbeispiel ist der Kanülen-Hub 29 an der Kanüle 19 angeformt, nämlich im Spitzgussverfahren hergestellt und an die Kanüle 19 angespitzt. Es ist aber auch ohne weiteres möglich, ihn an der Kanüle festzukleben. Das zweite Dichtelement 45 kann in den Kanülen-Hub eingelegt oder vorzugsweise eingespritzt sein, wobei dann der Kanülen-Hub 29 und das zweite Dichtelement 45 vorzugsweise in einem Zwei-Komponenten-Spritzguss-verfahren hergestellt werden.

Das erste Dichtelement 17 kann in die Kappe 3 eingesetzt werden oder aber durch ein Spritzgussverfahren hergestellt und damit in die Kappe 3 eingespritzt werden. Besonders bevorzugt werden die Kappe 3 und das erste Dichtelement 17 in einem Zwei-Komponenten-Spritzgussverfahren hergestellt und an die Kanüle 19 angespritzt.

Aus den Erläuterungen zu Figur 4 wird deutlich, dass hier die Kappe 3 und der Kanülen-Hub 29, der gegebenenfalls das zweite Dichtungselement 45 umfassen kann, getrennt hergestellt werden.

Bei der Herstellung des Aufsatzes 1 können also zunächst zwei getrennte Elemente hergestellt werden, nämlich der Kanülen-Hub 29, der vorzugsweise mit dem zweiten Dichtelement 45 versehen ist, und die Kappe 3 mit dem ersten Dichtungselement 17.

Bei der Herstellung des Kanülen-Hubs 29 wird vorzugsweise der Kanülen-Hub 29 an die Kanüle 19 angeformt, insbesondere angespritzt, während die Kanüle 19 an ihrem freien Ende festgehalten wird. Anschließend kann das zweite Dichtelement 45 in den Kanülen-Hub 29 eingesetzt werden. Vorzugsweise werden aber, sofern das zweite Dichtelement 45 vorgesehen ist, beide Teile, der Kanülen-Hub 29 und das zweite Dichtelement 45, in einem Zwei-Komponenten-Spritzgussverfahren hergestellt. Es wird hier deutlich, dass die Kanüle 19 sehr einfach auch in den Kanülen-Hub 29 eingeklebt werden kann.

Entsprechend wird die Kappe 3 vorzugsweise in einem Spritzgussverfahren hergestellt, wobei vorzugsweise das erste Dichtelement 17 und die Kappe 3 durch ein Spritzgussverfahren miteinander verbunden werden. Sofern die Kappe und das Dichtelement aus verschiedenen Materialien bestehen, was bevorzugt wird, werden die beiden Teile bevorzugt in einem Zwei-Komponenten-Spritzgussverfahren hergestellt.

Betrachtet man die in den Figuren 1 bis 3 dargestellten Ausführungsbeispiele und das in Figur 4 wiedergegebene Ausführungsbeispiel in seiner ganz rechts in Figur 4 dargestellten dritten Montageposition, so ergibt sich bezüglich der Herstellung des Aufsatzes 1 Folgendes:

Die Kanüle 19 kann an ihrem einer Injektion dienenden, in einem Abstand zur Spritze angeordnetem Ende erfasst werden. Das gegenüberliegende Ende kann in einem Kanülen-Hub 29 eingeformt werden. Der Kanülen-Hub 29 wird an dem freien Ende durch ein Spritzgussverfahren angeformt, wobei gleichzeitig der Kanülen-Hub 29 selbst hergestellt wird. Es ist auch möglich, die Kanüle in den Kanülen-Hub einzukleben.

Jetzt oder zu einem späteren Zeitpunkt kann in den Kanülen-Hub 29 ein zweites Dichtelement 45 eingelegt oder eingespritzt werden. Vorzugsweise wird jedoch vorgesehen, an dem freien Ende der Kanüle 19 in einem Zwei-Komponenten-Spritzgussverfahren sowohl den Kanülen-Hub 29 als auch das zweite Dichtelement 45 anzuformen, also beide Teile gleichzeitig herzustellen.

Das der Injektion dienende Ende der Kanüle 19 wird nun freigegeben. Die Kanüle 19 kann in einem Bereich im einem Abstand zu dem der Injektion dienenden Ende gehalten werden, oder aber am Kanülen-Hub 29. Nun kann an das jetzt freie Ende der Kanüle 19 ein erstes Dichtelement 17 angeformt, vorzugsweise angespritzt werden. Bei diesem Herstellungsverfahren ist es ausgeschlossen, dass die Kanülenspitze beschädigt wird. Dies kann sehr wohl beim Einstechen einer Kanüle in ein Dichtelement geschehen; die Spritze der Kanüle kann umgebogen werden und einen Widerhaken bilden.

Anschließend kann die Kappe 3 auf das erste Dichtelement 17 aufgesetzt werden. Vorzugsweise wird die Kappe 3 jedoch in einem Spritzgussverfahren angeformt, dabei an das erste Dichtelement 17 und an den Kanülen-Hub 29 angespritzt.

Besonders bevorzugt wird ein Verfahren, bei dem an das freie, der Injektion dienende Ende der Kanüle 19 in einem Zwei-Komponenten-Spritzgussverfahren das erste Dichtelement 17 und die komplette Kappe 3 angeformt, insbesondere angespritzt werden.

Der fertige, also vormontierte Aufsatz 1 kann nun auf eine Spritze 5 aufgesetzt werden, wobei bei Bedarf ein Haltering 25 verwendet wird, wie er anhand der Erläuterungen zu den Figuren 2 und 3 beschrieben wurde. Dabei kann der Haltering 25 zunächst am Ansatz 21 der Spritze 5 befestigt oder aber in das offene Ende des Aufsatzes 1 bzw. des Kanülen-Hubs 29 eingebracht werden. Schließlich kann der Haltering 25 auch als Teil des Kanülen-Hubs 29 realisiert sein.

Aus den Erläuterung zu den Figuren 1 bis 4 wird deutlich, dass das Herstellungsverfahren sehr einfach und variabel ist. Beispielsweise kann, sofern der Kanülen-Hub 29 aus einem weichen Material besteht auf ein zweites Dichtelement 45 verzichtet werden. Dieses kann aber ohne weiteres in den Kanülen-Hub 29 eingelegt oder vorzugsweise eingespritzt werden. Besonders bevorzugt werden, wie gesagt, der Kanülen-Hub 29 und das zweite Dichtelement 45 in einem Zwei-Komponenten-Spritzgussverfahren hergestellt.

Auch die Ausgestaltung der einzelnen Teile des Aufsatzes 1 kann in einem weitem Bereich frei gewählt werden. Es ist also möglich, Kappen 3 mit Stegen 15 herzustellen, wie sie anhand Figur 1 erläutert wurden. Da bei dieser Ausgestaltung die erste Dichtung 17 durch die Zwischenräume der Stege 15 von außen zugänglich ist, ergibt sich eine besonders gute Griffigkeit der Kappe 3 und des Kappenelements 9, was die Handhabbarkeit des Aufsatzes 1 verbessert, wenn für das erste Dichtelement 17 ein weiches griffiges Material wie Gummi oder TPE verwendet wird.

Die Herstellung anderer Ausgestaltungen der Kappe 3, beispielsweise einer becherförmigen, rundum geschlossenen Kappe mit konischer Außenfläche, ist ohne weiteres möglich. Eine derartige Ausgestaltung ist in Figur 4 wiedergegeben.

Es zeigt sich im Übrigen auch, dass der Kanülen-Hub unterschiedlich ausgeformt werden kann. Er kann also mit einer hier beschriebenen Grundplatte 33 versehen werden. Die davon ausgehende Wand 35 kann einzelne, nebeneinander liegende Haltearme aufweisen oder aber eine rundum geschlossene Wand 35. An dem der Spritze 5 im montiertem Zustand zugewandten Ende des Kanülen-Hubs 29 sind Verriegelungsvorrichtungen vorgesehen, hier wenigstens eine Rastnase 27, die den Kanülen-Hub 29 und damit den Aufsatz 1 sicher am Ansatz 21 einer Spritze 5 halten. Ein Vergleich der Ausgestaltung der in den Figuren 1 bis 3 und 4 dargestellte Ansätze zeigt, das der Aufsatz 1 mit unterschiedlich ausgestatteten Spritzen kombiniert werden kann.

Durch die Wahl der Materialien für die Kappe 3, den Kanülen-Hub 29, das erste Dichtelement 17 und für das vorzugsweise vorgesehene zweite Dichtelement 45 können die Eigenschaften des Aufsatzes 1 and die Anforderungen, an die Handhabung und an die Dichtung gegenüber Kanüle 19 und Spritze 5 in einem weiten Rahmen angepasst werden. Es können also harte und weiche Materialien kombiniert und gemeinsam in einem Zwei-Komponenten-Spritzgussverfahren verarbeitet werden.

Bei entsprechender Wahl des Materials für den Kanülen-Hub 29 ist es auch möglich, nach Anbringung des Kanülen-Hubs 29 die Kanüle 19 zu silikonisieren. Es wird hier also ein Aufsatz 1 geschaffen, der an unterschiedliche Verwendungszwecke anpassbar ist.

Durch das Anformen des ersten Dichtelements 17 an das der Injektion dienende Ende der Kanüle 19 wird sichergestellt, dass dieses nicht beschädigt wird, insbesondere vermieden, das hier Widerhaken entstehen. Darüber hinaus wird ein Verbiegen der Kanüle 19 mit hoher Sicherheit vermieden.

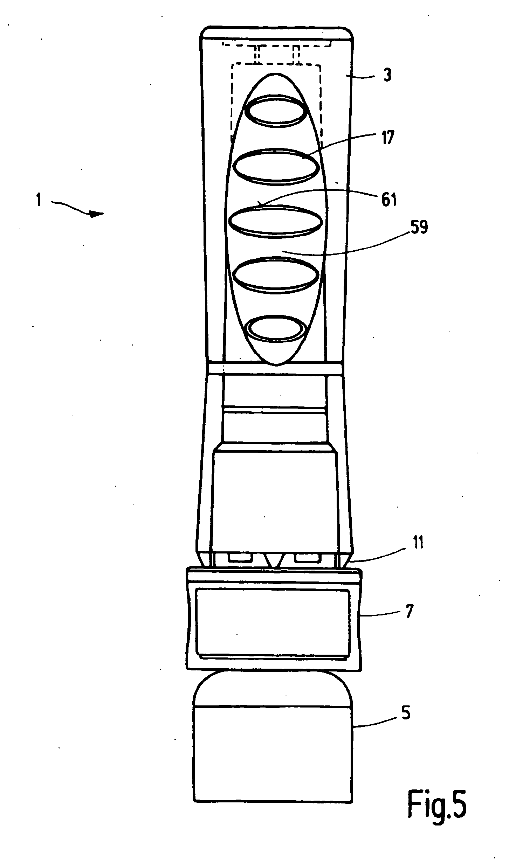

Figur 5 zeigt ein abgewandeltes Ausführungsbeispiel eines Aufsatzes 1 in Seitenansicht. Er weist eine Kappe 3 auf, die vorzugsweise aus einem harten Kunststoff besteht und ein Dichtelement 17 umschließt, das aus Gummi oder vorzugsweise TPE besteht oder-zumindest eines oder beide dieser Materialien umfasst. Bei dem hier dargestellten Ausführungsbeispiel ist die Kappe 3 über eine Sollbruchlinie 11 mit einem Haltebereich 7 verbunden, der an einer Spritze 59 angebracht ist. Es ist sehr wohl möglich, den Aufsatz 1 ohne eine derartige Sollbruchlinie 1 auszubilden und unmittelbar auf der Spritze 5 zu befestigen.

Die Kappe 3 ist im Wesentlichen zylindrisch ausgebildet. Sie weist an ihrer Umfangsfläche eine, vorzugsweise zwei gegenüberliegende Abflachungen 59 auf, die die Griffigkeit der Kappe 3 verbessern. Bei dem hier dargestellten Ausführungsbeispiel ist die Kappe 3 im Bereich der Abflachungen 59 mit mindestens einem Durchbruch 61 versehen, über den das Dichtelement 17 beim Erfassen der Kappe 3 mit den Fingern des Benutzers des Aufsatzes 1 in Berührung kommt. Da das Dichtelement 37, wie anhand der oben beschriebenen Ausführungsbeispiele deutlich wurde, aus Gummi oder vorzugsweise TPE beseht, wird dadurch die Griffigkeit der Kappe 3 wesentlich gesteigert.

Bei dem hier dargestellten Ausführungsbeispiel sind mehrere übereinanderliegende Durchbrüche 61 vorgesehen, die in Draufsicht elliptisch ausgebildet sind, wobei die größere Durchmesserlinie quer zur Längsachse des Aufsatzes 1 beziehungsweise der Kappe 3 verläuft. Da die Abflachung 59 über ihre Länge gesehen, also über die Höhe gemäß Figur 5, eine zunächst zunehmende und dann abnehmende Tiefe aufweist, ist die Breite der Durchbrüche 61 über die Höhe der Abflachung 59 gesehen nicht gleich, vielmehr in der Mitte größter als oben und unten, weil die Abflachung 59 symmetrisch ausgebildet ist.

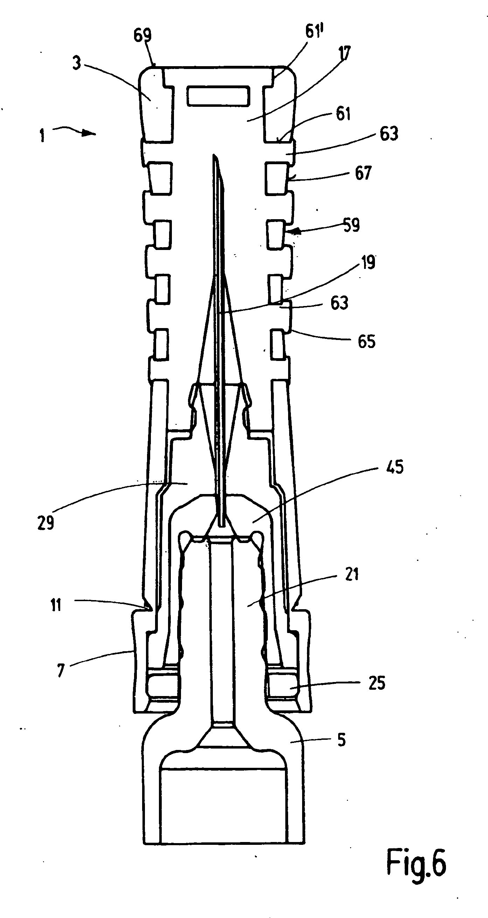

Figur 6 zeigt den Aufsatz 1 gemäß Figur 5 im Längsschnitt. Es ist ersichtlich, dass er eine Kappe 3 umfasst, die über die Sollbruchlinie L in den Haltebereich 7 übergeht. Dieser ist, wie anhand von Figur 2 erläutert, an einem Ansatz 21 der Spritze 5 über einen Haltering 25 fixiert. Dieser hält einen Kanülen-Hub 29, in den eine Kanüle 19 vorzugsweise eingeklebt ist. Denkbar ist es auch, den Kanülen-Hub 29 an der Kanüle 19 anzuformen, insbesondere anzuspritzen.

Der Kanülen-Hub 29 umgreift den Ansatz 21 der Spritze 5 kappenartig. In das Innere des Kanülen-Hubs ist hier, wie anhand von Figur 3 erläutert, ein zweites Dichtelement 45 eingebracht, vorzugsweise eingespritzt, das aus Gummi oder TPE besteht beziehungsweise diese Substanzen umfasst. Das zweite Dichtelement 45 umfasst die Umfangsfläche des Ansatzes 21 und liegt auf dessen Stirnseite dichtend auf, sodass hier ein optimaler Abschluss gewährleistet ist

In das obere Ende der Kappe 3, das dem Ansatz 21 beziehungsweise dem Haltebereich 7 gegenüberliegt, ist das erste Dichtelement 17 eingespritzt. Es wird hier deutlich, dass das Material des ersten Dichtelements 17 die Durchbrüche 61 durchdringt, wobei Stege ausgebildet werden, die einen Formschluss zwischen Kappe 3 und Dichtelement 17 gewährleisten. Durch einen Kreis ist angedeutet, dass die Stege in ihren Endbereichen 65 die Außenfläche 67 der Kappe 3, hier der Abflachung 59 etwas überragen. Die Stege 63 sind im Querschnitt gesehen quasi T-förmig ausgebildet. Dies führt einerseits zu einer sicheren Verbindung zwischen Kappe 3 und Dichtelement 17, andererseits dazu, dass beim Erfassen der Kappe 3 die durch das Dichtelement 17 gebildete Fläche etwas vergrößert wird, also größer ist als die zugehörigen Durchbrüche 61. Bei dem hier dargestellten Ausführungsbeispiel ist auch im Bereich der Stirnseite 69 ein Durchbruch 61' vorgesehen, in dessen Bereich das erste Dichtelement 17 bis an die Stirnseite 69 reicht, also einen Teil der Außenfläche der Kappe 3 bildet.

Diese Ausgestaltung dient dazu, einen möglichst großen Bereich der Außenfläche der Kappe 3 durch das erste Dichtelement 17 zu realisieren, wie dies auch bereits bei dem Ausführungsbeispiel gemäß Figur 1 vorgesehen ist. Da insbesondere TPE dampfdurchlässig ist, wird, wenn dieses Material verwendet wird, durch die große Außenfläche des ersten Dichtelements 17 erreicht, dass eine optimale Endsterilisierung beziehungsweise Terminalsterilisierung des Aufsatzes 1 möglich ist: Der Dampf kann bis in das Innere des Aufsatzes 1 eindringen, sodass auch hier eine optimale Sterilisierung gewährleistet ist.

Da das erste Dichtelement 17 aus Gummi beziehungsweise vorzugweise aus TPE besteht, wird die Kanüle 19 dicht abgeschlossen, sodass eine mikrobiologische Verunreinigung des Inhalts der Spritze 5 praktisch ausgeschlossen werden kann. Der unmittelbare Auslass der Spritze 5 im Bereich des Ansatzes 21 wird durch das zweite Dichtelement 45 bei dem hier dargestellten Ausführungsbeispiel abgedichtet.

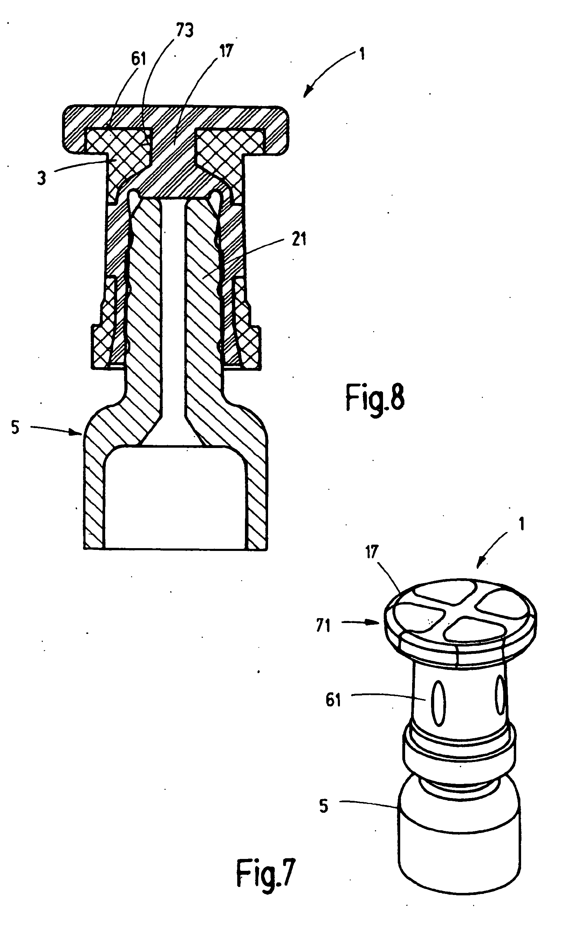

Figur 7 zeigt einen nicht zur Erfindung gehörenden Aufsatz 1, der einen zylindrischen Grundkörper aufweist, der einen Ansatz einer Spritze 5 umgibt und in einem verbreiterten Kopf 71 an seinem der Spritze 5 abgewandten Ende endet.

Auch hier sind mindestens ein, vorzugsweise mehrere über die Umfangsfläche des Aufsatzes 1 verteilte Durchbrüche 61 vorgesehen, die mit dem Material des ersten Dichtelements 17 ausgefüllt sind. Dieses reicht also hier bis zur Außenfläche des Aufsatzes 1. Durch dunklere Bereiche des Kopfes 71 wird angedeutet, dass auch hier das Material des ersten Dichtelements 17 bis zur Außenfläche des Aufsatzes 1 reicht. Dies führt dazu, dass die Griffigkeit des Aufsatzes 1 verbessert wird, andererseits dazu, dass Teile der Außenfläche des Aufsatzes 1 durch das dampfdurchlässige Material des Dichtelements 17 gebildet werden, sodass Dampf in das Innere des Aufsatzes 1 eindringen und zur Sterilisierung des Aufsatzes 1 auf der Spritze 5 beitragen kann.

Anhand von Figur 8, die einen Längsschnitt durch den Aufsatz gemäß Figur 7 zeigt, wird deutlich, dass das Dichtelement 17 den Ansatz 21 der Spritze 5 umgibt und das Innere der Kappe 3 ausfüllt. Deutlich wird auch, dass das Material des Dichtelements 17 bis zur Außenfläche der Kappe 3 reicht, um, wie erläutert, die Griffigkeit des Aufsatzes 1 zu erhöhen und die Sterilisierung zu optimieren.

Bei dem hier dargestellten Aufsatz ist im Bereich der Stirnseite 69 der Kappe 3 eine zentrale Öffnung 73 vorgesehen, über die das Material des Dichtelements 17 nach oben aus dem Innenraum der Kappe 3 ragt und die Außenfläche der Kappe 3 bilden kann. Aus Figur 7 wird deutlich, dass das Dichtelement 17 hier beispielsweise zwei senkrecht aufeinanderstehende, entlang einer Durchmesserlinie verlaufende Stege bildet, die sich im Bereich der Umfangsfläche des Kopfes 71 verbreitern, um die Griffbereiche zu vergrößern.

Die Anzahl der Stege und Griffbereiche sowie die der Durchbrüche kann in einem weiten Rahmen gewählt werden.

Wesentlich ist hier wie bei allen anderen Ausführungsbeispielen des Aufsatzes 1, dass das Dichtelement 17 vorzugsweise in die Kappe 3 eingespritzt wird, um eine sichere Verbindung zwischen Kappe und Dichtelement zu gewährleisten, außerdem um eine optimale mikrobiologische Abdichtung der Spritze 5 zu gewährleisten.

Vorzugsweise ist stets vorgesehen, dass die Kappe 3 aus einem harten Kunststoff besteht beziehungsweise diesen Kunststoff umfasst, und das Dichtelement 17, wie auch das zweite Dichtelement 45, aus einem weicheren Material, vorzugsweise Gummi oder insbesondere TPE. TPE wird vorzugsweise mit einer Härte von 30 bis 60 Shore A gewählt. Dadurch wird einerseits eine optimale Anpassungsfähigkeit der Dichtelemente an der Oberfläche der abzudichtenden Bereiche gewährleistet, andererseits eine ausreichende Stabilität und Griffigkeit des Aufsatzes 1.

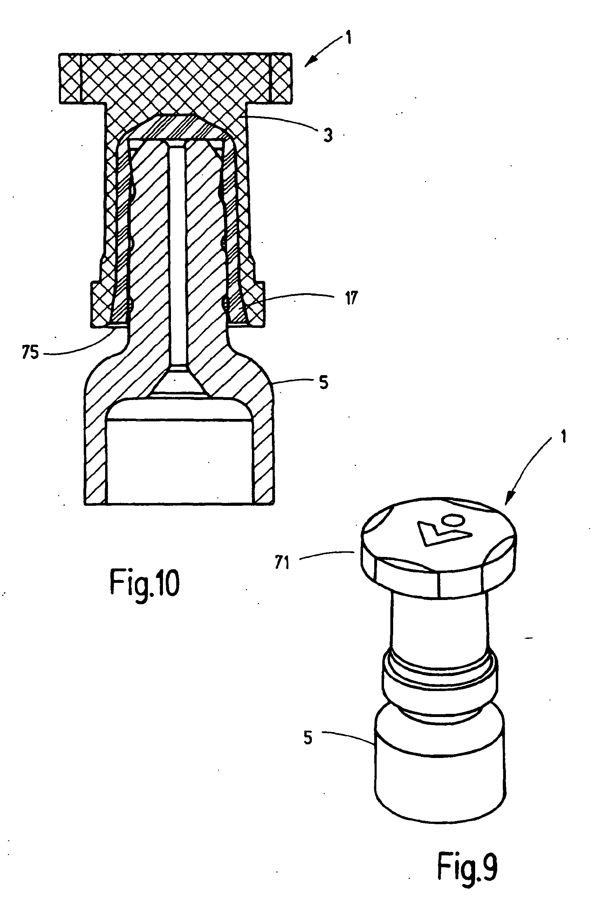

Figur 9 zeigt einen weiteren, nicht zur Erfindung gehörenden Aufsatz 1, der auf eine Spritze 5 aufgebracht ist. Auch hier ist, wie in Figur 7, ein quasi pilzförmiger Aufsatz 1 vorgesehen, dessen zylindrischer Grundkörper einen Ansatz der Spritze 5 umgibt und der einen an seinem der Spritze abgewandten Ende einen Kopf 71 aufweist. Auch hier ist der Außenbereich des Kopfes mit dem Material des Dichtelements versehen, um die Griffigkeit des Aufsatzes 1 zu verbessern.

Die Schnittdarstellung gemäß Figur 10 zeigt, dass der Aufsatz 1 eine Kappe 3 aufweist, die rundum geschlossen ist und das erste Dichtelement 17 einschließt. Die Materialwahl ist hier wie bei den oben genannten Ausführungsbeispielen vorgenommen worden: die Kappe 1 besteht vorzugsweise aus Hartkunststoff, während das Dichtelement 17 aus Gummi oder vorzugsweise TPE mit der vorgegebenen Härte besteht.

Die mit dem Material des Dichtelements versehenen Abschnitte im Bereich des Kopfes 71 stehen mit dem Innenraum der Kappe 3 hier nicht in Verbindung, sodass bei einer Sterilisierung der Spritze 5 der Dampf lediglich über die der Spritze 5 zugewandte Unterkante 75 des Dichtelements 17 in das Innere der Kappe 3 vordringen kann und zur optimalen Sterilisierung des Aufsatzes 1 beiträgt.

Es hat sich gezeigt, dass die Verbindung zwischen Kappe und Dichtelement mittels eines Spritzgussverfahrens besonders einfach realisierbar ist. Da die Kappe und das Dichtelement unterschiedliche Aufgaben haben, werden vorzugsweise unterschiedliche Materialien gewählt: die Kappe weist Hartkunststoff auf oder besteht aus diesem Material, um das weichere Dichtelement zu schützen und zu stützen. Die beiden Materialien werden vorzugsweise so ausgewählt, dass sie in einem Zwei-Komponenten-Spritzgussverfahren eingesetzt werden, um den Aufsatz 1 zu realisieren. Es hat sich gezeigt, dass nicht in allen Fällen eine optimale Verbindung zwischen Kappe und Dichtelement realisierbar ist. Daher wird besonders bevorzugt die Kappe 3 so ausgestaltet, dass das Dichtelement in deren Inneren durch Formschluss gehalten wird.

Dies gilt im Übrigen auch für die Kombination des zweiten Dichtelements 45 und des Kanülen-Hubs 29. Letzterer besteht aus einem Hartkunststoff, der die Kanüle 19 trägt und der das zweite Dichtelement 45 aufnimmt. Dieses besteht aus einem weicheren Material, vorzugsweise aus Gummi oder TPE, um die gewünschten Dichtungseigenschaften zu gewährleisten. Außerdem zeichnet sich das vorzugsweise eingesetzte TPE durch eine gute Dampfdurchlässigkeit aus, sodass der fertig montierte Aufsatz 1 gut sterilisierbar ist.

Bei der Realisierung eines Formschlusses zwischen Kappe und Dichtelement kann, wie anhand der Kappe 3 und dem ersten Dichtelement 17 erläutert, vorgesehen werden, dass die Kappe Durchbrechungen aufweist, durch die das Material des ersten Dichtelements zumindest bis zur Außenfläche der Kappe 3 vordringt, vorzugsweise diese etwas übergreift, was anhand von Figur 6 besonders deutlich wird.

Figur 6 zeigt im Übrigen, dass das Grundprinzip des Einspritzens von Dichtmaterial in eine Kappe sowohl im Zusammenhang mit der Kappe 3 und dem ersten Dichtelement 17 als auch im Zusammenhang mit dem kappenförmigen Kanülen-Hub 29 und dem zweiten Dichtelement 45 realisiert werden kann, wobei vorzugsweise ein Zwei-Komponenten-Spritzgussverfahren eingesetzt wird, weil dieses die gleichzeitige Herstellung von Kappe und Dichtelement ermöglicht.

## Patentansprüche

1. Aufsatz für eine Spritze oder Karpule mit einer Kanüle, wobei der Aufsatz ein Dichtelement umfasst, wobei das Dichtelement (17) auf die Kanüle (19) aufgespritzt ist, **dadurch gekennzeichnet, dass** eine Kappe (3) vorgesehen ist, die auf das Dichtelement aufspritzbar ist.

2. Aufsatz für eine Spritze oder Karpule mit einem Grundkörper und einem Ansatz zur Befestigung des Aufsatzes, mit einer Kappe und mit einem Dichtelement, wobei das Dichtelement (17) und die Kappe (3) durch ein Spritzgussverfahren miteinander verbunden sind, **dadurch gekennzeichnet, dass** ein Kanülen-Hub (29) vorgesehen ist, in den eine Kanüle (19) eingesetzt ist und der Gummi oder TPE umfasst oder aus Gummi oder TPE besteht.

3. Aufsatz nach Anspruch 1 oder 2, **dadurch gekennzeichnet, dass** das Dichtelement (17) und die Kappe (3) in einem Zwei-Komponenten-Spritzgussverfahren herstellbar sind.

4. Aufsatz nach einem der vorhergehenden Ansprüche, **dadurch gekennzeichnet, dass** das Dichtelement (17) TPE und/oder Gummi umfasst, vorzugsweise aus TPE und/oder Gummi besteht.

5. Aufsatz nach einem der vorhergehenden Ansprüche 1 bis 4, **dadurch gekennzeichnet, dass** die Kappe (3) Hartkunststoff umfasst, vorzugsweise aus Hartkunststoff besteht.

6. Aufsatz nach einem der vorhergehenden Ansprüche 1 bis 5, **dadurch gekennzeichnet, dass** die Kappe (3) mindestens eine Ausnehmung aufweist, durch die das Dichtelement (17) von außen zugänglich ist.

7. Aufsatz nach einem der vorhergehenden Ansprüche 1 bis 6, **dadurch gekennzeichnet, dass** das Dichtelement (17) die Außenfläche der Kappe (3) überragt.

8. Aufsatz nach einem der vorhergehenden Ansprüche 1 bis 7, **gekennzeichnet durch** einen zwischen Kappe (3) und Dichtelement (17) gegebenen Formschluss.

9. Aufsatz nach einem der Ansprüche 2 bis 8, **dadurch gekennzeichnet, dass** die Kanüle (19) in den Kanülen-Hub (29) eingeklebt oder eingeformt ist.

10. Aufsatz nach einem der Ansprüche 2 bis 8, **dadurch gekennzeichnet, dass** die Kanüle (19) in den Kanülen-Hub (29) eingespritzt ist.

11. Aufsatz nach einem der vorhergehenden Ansprüche 2 bis 10, **dadurch gekennzeichnet, dass** der Kanülen-Hub (29) Kunststoff umfasst oder aus Kunststoff besteht.

12. Aufsatz nach einem der vorhergehenden Ansprüche 2 bis 11, **dadurch gekennzeichnet, dass** er ein Dichtelement (45) aufweist, das Gummi oder TPE umfasst oder aus Gummi oder aus TPE besteht und auf der der Kanüle abgewandten Seite des Kanülen-Hubs (29) angeordnet ist.

13. Aufsatz nach Anspruch 12, **dadurch gekennzeichnet, dass** das Dichtelement (45) in den Kanülen-Hub (29) eingespritzt ist.

14. Aufsatz nach Anspruch 13, **dadurch gekennzeichnet, dass** der Kanülen-Hub (29) und das Dichtelement (45) im Zwei-Komponenten-Spritzgussverfahren herstellbar sind.

15. Verfahren zur Herstellung eines Aufsatzes für eine Spritze oder Karpule mit folgenden Schritten:
- Erfassen einer Kanüle (19) an ihrem Ende, das nicht zur Injektion dient,
- Umspritzen des Endes der Kanüle (19), das zur Injektion dient, mit einem Dichtelement (17),
**dadurch gekennzeichnet, dass**
- das Dichtelement mit einer Kappe (3) umspritzt wird.

16. Verfahren zur Herstellung eines Aufsatzes nach Anspruch 1 oder einem der Ansprüche 3 bis 8 bezogen auf Anspruch 1, **dadurch gekennzeichnet, dass** das Dichtelement (17) und die Kappe (3) durch ein Spritzgussverfahren miteinander verbunden sind.

17. Verfahren zur Herstellung eines Aufsatzes nach Anspruch 2 oder einem der Ansprüche 3 bis 14 besogen auf Anspruch 2 wobei das Dichtelement (17) und die Kappe (3) mittels eines Spritzgussverfahrens miteinander verbunden werden **dadurch gekennzeichnet, dass** ein Kanülen-Hub (29) vorgesehen ist, in den eine Kanüle (19) eingesetzt ist und der Gummi oder TPE umfasst oder aus Gummi oder TPE besteht.

18. Verfahren nach einem der Ansprüche 16 oder 17, **dadurch gekennzeichnet, dass** das Dichtelement (17) und die Kappe (3) mittels eines Zwei-Komponenten-Spritzgussverfahrens miteinander verbunden werden.

19. Verfahren nach einem der Ansprüche 16 oder 17, **dadurch gekennzeichnet, dass** die Kappe (3) und das Dichtelement (17) unmittelbar an die Kanüle angespritzt werden.

20. Spritze oder Karpule mit einem Aufsatz nach einem der Ansprüche 1 bis 14.

21. Spritze oder Karpule mit einem Aufsatz, der nach einem Verfahren gemäß einem der Ansprüche 15 bis 19 hergestellt ist.

## Claims

1. An attachment for a syringe or cartridge having a cannula, the attachment comprising a sealing element, the sealing element (17) being molded onto the cannula (19), **characterized in that** a cap (3) is provided, which can be molded onto the sealing element.

2. An attachment for a syringe or cartridge, having a base body and a projection for fastening the attachment, having a cap and a sealing element, the sealing element (17) and the cap (3) being connected to one another by means of an injection molding method, **characterized in that** a cannula lift (29) is provided, into which a cannula (19) is inserted and which comprises rubber or TPE, or is made of rubber or TPE.

3. The attachment according to claim 1 or 2, **characterized in that** the sealing element (17) and the cap (3) can be produced using a two-component injection molding method.

4. An attachment according to one of the preceding claims, **characterized in that** the sealing element (17) comprises TPE and/or rubber, and preferably is made or TPE and/or rubber.

5. An attachment according to one of the preceding claims 1 to 4, **characterized in that** the cap (3) comprises hard plastic, and preferably is made of hard plastic.

6. An attachment according to one of the preceding claims 1 to 5, **characterized in that** the cap (3) comprises at least one recess by which the sealing element (17) is accessible from the outside.

7. An attachment according to one of the preceding claims 1 to 6, **characterized in that** the sealing element (17) projects beyond the outer surface of the cap (3).

8. An attachment according to one of the preceding claims 1 to 7, **characterized by** positive fit provided between the cap (3) and sealing element (17).

9. The attachment according to one of the claims 2 to 8, **characterized in that** the cannula (19) is glued or formed into the cannula lift (29).

10. The attachment according to one of the claim 2 to 8, **characterized in that** the cannula (19) is molded into the cannula lift (29).

11. An attachment according to one of the preceding claims 2 to 10, **characterized in that** the cannula lift (29) comprises plastic or is made of plastic.

12. An attachment according to one of the preceding claims 2 to 11, **characterized in that** it has a sealing element (45), which comprises rubber or TPE, or is made of rubber or TPE, and which is disposed on the side of the cannula lift (29) facing away from the cannula.

13. The attachment according to claim 12, **characterized in that** the sealing element (45) is molded into the cannula lift (29).

14. The attachment according to claim 13, **characterized in that** the cannula lift (29) and the sealing element (45) can be produced using a two-component injection molding method.

15. A method for producing an attachment for a syringe or cartridge, comprising the following steps:
- seizing a cannula (19) at its end not serving injection purposes,
- molding a sealing element (17) around the end of the cannula (19) serving injection purposes,
**characterized in that**
- a cap (3) is molded around the sealing element.

16. A method for producing an attachment according to claim 1 or one of claims 3 to 8 related to claim 1, **characterized in that** the sealing element (17) and the cap (3) are connected to one another by means of an injection molding method.

17. A method for producing an attachment according to claim 2 or one of claims 3 to 14 related to claim 2, wherein the sealing element (17) and the cap (3) are connected to one another by means of a injection molding method, **characterized in that** a cannula lift (29) is provided, into which a cannula (19) is inserted and which comprises rubber or TPE, or is made of rubber or TPE.

18. The method according to one of the claims 16 or 17, **characterized in that** the sealing element (17) and the cap (3) are connected to one another by means of a two-component injection molding method.

19. The method according to one of the claims 16 or 17, **characterized in that** the cap (3) and the sealing element (17) are molded directly onto the cannula.

20. A syringe or cartridge having an attachment according to one of the claims 1 to 14.

21. A syringe or cartridge having an attachment, which is produced by a method according to any one of the claims 15 to 19.

## Revendications

1. Raccord pour une seringue ou carpule présentant une canule, dans lequel le raccord comprend un élément d'étanchéité, l'élément d'étanchéité (17) étant moulé par injection sur la canule (19), **caractérisé en ce qu'**un capuchon (3) est prévu qui peut être moulé par injection sur l'élément d'étanchéité.

2. Raccord pour une seringue ou carpule présentant une base et une attache pour fixer le raccord, avec un capuchon et un élément d'étanchéité, l'élément d'étanchéité (17) et le capuchon (3) étant raccordés l'un à l'autre par un procédé de moulage par injection, **caractérisé en ce qu'**une levée de canule (29) est prévue, dans laquelle une canule (19) est insérée et qui comprend du caoutchouc ou du TPE ou se compose de caoutchouc ou de TPE.

3. Raccord selon la revendication 1 ou 2, **caractérisé en ce que** l'élément d'étanchéité (17) et le capuchon (3) peuvent être fabriqués au cours d'un procédé de moulage par injection à deux composants.

4. Raccord selon l'une des revendications précédentes, **caractérisé en ce que** l'élément d'étanchéité (17) comprend du TPE et/ou du caoutchouc, de préférence se compose de TPE et/ou de caoutchouc.

5. Raccord selon l'une des revendications précédentes 1 à 4, **caractérisé en ce que** le capuchon (3) comprend du plastique rigide, de préférence se compose de plastique rigide.

6. Raccord selon l'une des revendications 1 à 5, **caractérisé en ce que** le capuchon (3) présente au moins un évidement par lequel il est possible d'accéder à l'élément d'étanchéité (17) de l'extérieur.

7. Raccord selon l'une des revendications précédentes 1 à 6, **caractérisé en ce que** l'élément d'étanchéité (17) fait saillie par rapport à la surface extérieure du capuchon (3).

8. Raccord selon l'une des revendications précédentes 1 à 7, **caractérisé par** un procédé d'accouplement mécanique donné entre le capuchon (3) et l'élément d'étanchéité (17).

9. Raccord selon l'une des revendications 2 à 8, **caractérisé en ce que** la canule (19) est encollée ou moulée dans la levée de canule (29).

10. Raccord selon l'une des revendications 2 à 8, **caractérisé en ce que** la canule (19) est moulée par injection dans la levée de canule (29).

11. Raccord selon l'une des revendications précédentes 2 à 10, **caractérisé en ce que** la levée de canule (29) comprend du plastique ou se compose de plastique.

12. Raccord selon l'une des revendications précédentes 2 à 11, **caractérisé en ce qu'**il présente un élément d'étanchéité (45), qui comprend du caoutchouc ou du TPE ou se compose de caoutchouc ou de TPE et est agencé sur le côté de la levée de canule (29) opposé à la canule.

13. Raccord selon la revendication 12, **caractérisé en ce que** l'élément d'étanchéité (45) est moulé par injection dans la levée de canule (29).

14. Raccord selon la revendication 13, **caractérisé en ce que** la levée de canule (29) et l'élément d'étanchéité (45) peuvent être fabriqués au cours d'un procédé de moulage par injection à deux composants.

15. Procédé de fabrication d'un raccord pour une seringue ou carpule comportant les étapes suivantes :
- saisie d'une canule (19) au niveau de son extrémité qui ne sert pas à l'injection,
- extrusion de l'extrémité de la canule (19) qui sert à l'injection avec un élément d'étanchéité (17),
**caractérisé en ce que**
- l'élément d'étanchéité est extrudé avec un capuchon (3).

16. Procédé de fabrication d'un raccord selon la revendication 1 ou l'une des revendications 3 à 8 en référence à la revendication 1, **caractérisé en ce que** l'élément d'étanchéité (17) et le capuchon (3) sont raccordés l'un à l'autre par un procédé de moulage par injection.

17. Procédé de fabrication d'un raccord selon la revendication 2 ou l'une des revendications 3 à 14 en référence à la revendication 2, dans lequel l'élément d'étanchéité (17) et le capuchon (3) sont raccordés l'un à l'autre au moyen d'un procédé de moulage par injection, **caractérisé en ce qu'**une levée de canule (29) est prévue, dans laquelle une canule (19) est insérée et qui comprend du caoutchouc ou du TPE ou se compose de caoutchouc ou de TPE.

18. Procédé selon l'une des revendications 16 ou 17, **caractérisé en ce que** l'élément d'étanchéité (17) et le capuchon (3) sont raccordés l'un à l'autre au moyen d'un procédé de moulage par injection à deux composants.

19. Procédé selon l'une des revendications 16 ou 17, **caractérisé en ce que** le capuchon (3) et l'élément d'étanchéité (17) sont moulés par injection directement sur la canule.

20. Seringue ou carpule présentant un raccord selon l'une des revendications 1 à 14.

21. Seringue ou carpule présentant un raccord qui est fabriqué suivant un procédé selon l'une des revendications 15 à 19.
